Europäisches Patentamt

European Patent Office

Office européen des brevets

(19)

(11) Publication number: **0 016 573**
**B1**

(12) **EUROPEAN PATENT SPECIFICATION**

(45) Date of publication of patent specification: **13.06.84**  (51) Int. Cl.³: **C 12 Q 1/00**

(21) Application number: **80300652.7**

(22) Date of filing: **04.03.80**

(54) Enzyme reference composition.

(30) Priority: **05.03.79 US 17871**
**21.01.80 US 114009**

(43) Date of publication of application:
**01.10.80 Bulletin 80/20**

(45) Publication of the grant of the patent:
**13.06.84 Bulletin 84/24**

(84) Designated Contracting States:
**DE FR GB IT SE**

(56) References cited:
**US - A - 3 551 296**
**US - A - 3 629 142**
**US - A - 3 876 375**
**US - A - 3 928 137**
**US - A - 4 121 905**

(73) Proprietor: **BECKMAN INSTRUMENTS, INC.**
**2500 Harbor Boulevard**
**Fullerton California 92634 (US)**

(72) Inventor: **Louderback, Allan L.**
**9661 Longden Avenue**
**Temple City California (US)**

(74) Representative: **Wotherspoon, Graham et al,**
**FITZPATRICKS 4 West Regent Street**
**Glasgow G2 1RS Scotland (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### 1. Field of the invention

This invention relates to a laboratory material and, more particularly, to a stable enzyme reference composition.

### 2. Description of the prior art

Various compositions capable of being employed in conjunction with the analysis of enzymes to either calibrate an instrument or to periodically verify that the instrument is still operating within the tolerances desired are known to those skilled in the art.

For example, U.S.—A—3,466,249 (hereinafter referred to as Anderson) discloses a blood serum reference standard comprising a first container of freeze-dried blood serum and a second container of aqueous ammonium bicarbonate. The final pH of Anderson's reconstituted serum is 7.5±0.5. This pH range is felt by Anderson to be within an acceptable range for test systems as well as for stability of the reconstituted serum's components, particularly the enzymes.

U.S.—A—3,629,142 (hereinafter referred to as Marbach) discloses a freeze-dried serum reference standard comprising blood serum and a carbonate or bicarbonate of tris(hydroxymethyl)-aminomethane which is reconstituted by the addition of distilled water. The tris carbonate imparts a normal pH to the serum so as to provide for the stability of its constituents, and particularly the enzymes. (For normal serum and plasma that are collected under routine conditions, the normal pH range is generally considered to be about 7.3 to about 7.45. See Tietz, Fundamental of Clinical Chemistry, W. B. Saunders Co., Philadelphia, PA. (1970), pg. 634; and Henry et al, Clinical Chemistry, Principles and Technics, 2nd Edition, Harper & Row, New York, NY (1974), pgs. 774—778).

U.S.—A—3,876,375 and 4,121,905 (hereinafter referred to Maurukas I and Maurukas II) disclose a biological reference control composition comprising in its non-biological compound from about 60 to about 80 weight percent water and from about 20 to about 40 weight percent of at least one alkylene polyol having from 2 to 5 carbon atoms, the remainder being chiefly at least one natural biological material selected from the group consisting of blood serum, enzyme, metabolites, electrolytes, and hormones. Although the pH of this composition is not disclosed in either Maurukas I or Maurukas II, experiments have shown the pH to be from about 8.3 to about 8.5.

One short coming of the above prior art composition is that their enzyme constituents are highly unstable. For example, the manufacturer of a commercial composition believed to be within the scope of Marbach, supra, recommends that determinations of enzyme constituents of its distilled or deionized water reconstituted serum be made only on the day that the serum is reconstituted.

Similarly, the manufacturer of a commercial composition within the scope of Anderson also recommends that the determinations of its reconstituted composition be made only on the day that the serum is reconstituted.

Both of the above reconstituted compositions are recommended by their manufacturer to be stored between 2° and 8°C.

### Summary of the invention

The instant invention encompasses an enzyme reference composition having an improved shelf life. The composition comprises at least one enzyme of known value; 20 to 40 weight percent of at least one alkylene polyol having from 2 to 5 carbon atoms; 3 to 8 gm/dl total protein present is a human serum albumin matrix; and 60 to 80 weight percent water.

The enzyme constituents of a composition within the scope of this invention retain 90% of their initial value, based upon an Arrhenius Plot, for the periods set forth in Table I.

TABLE I
Arrhenius plot for 90% life, at 4°C storage

| Enzyme constitution | Shelf-life |
| --- | --- |
| Amylase | 6.8 Years |
| $\gamma$-Glutamyl transpeptidase | 206.9 Years |
| Lactic dehydrogenase | 1.0 Years |
| Creatine kinase | 145.9 Years |
| Alkaline phosphatase | 9.9 Months |
| Aspartate amino transferase | 2.4 Years |
| Alanine amino transferase | 6.6 Years |

The above Arrhenius plot shows that the enzyme constituents of the enzyme reference compositions within the scope of this invention are stable for periods of time far exceeding the shelf life of enzyme constituents present in prior art reference compositions.

2

Description of the preferred embodiments

The enzyme reference composition of the instant invention comprises at least one enzyme constituent of known value. Any enzyme of clinical significance can be present therein. Typical enzymes which are currently assayed in clinical laboratories include acid phosphatase (ACP); aldolase; alkaline phosphatase (ALP); amylase; cholinesterase; creatine kinase (CK; also known as creatine phosphokinase (CPK)); $\alpha$-glutamyl transpeptidase ($\gamma$-GT; GGT); $\alpha$-hydroxybutyric dehydrogenase ($\alpha$-HBD; HBD); isocitric dehydrogenase (ICD); lactic dehydrogenase (LDH); leucine aminopeptidase (LAP); lipase; alanine amino transferase (SGPT; ALT; GPT; glutamic-pyruvic transaminase); and aspartate amino transferase (SGOT; AST: GOT: glutamic-oxalacetic transaminase).

The activity of each enzyme present in the enzyme reference composition is not critical. Preferably, the activity of each enzyme should be within a range covering both normal and abnormal values.

The source of each enzyme is also not critical. However, the preference source of several enzymes are set forth in Table II.

TABLE II

| Enzyme constituent | Preferred source |
| --- | --- |
| ACP | Human seminal fluid |
| ALP | Calf intestine |
| Amylase | Pig pancreas |
| CK | Monkey muscle |
| SGOT | Pig heart |
| SGPT | Pig heart |
| $\gamma$-GT | Pig intestine |
| LDH | Chicken heart |

The enzyme reference composition of the instant invention also comprises from 20 to 40 weight percent of at least one alkylene polyol having from 2 to 5 carbon atoms. Preferably, the alkylene polyol comprises from 30 to 34 weight percent of the enzyme reference composition.

Suitable alkylene polyols which can be used include, but are not limited to, ethylene glycol, propylene glycol, butylene glycol, pentanediol, and glycerol. The alkylene polyol material is preferably selected from a group consisting of ethylene glycol, propylene glycol, glycerol, and mixtures thereof.

The enzyme reference composition of the instant invention also comprises from 3 to 8 gm/dl total protein. Preferably, from 4 to 5 gm/dl total protein is present in the enzyme reference composition. The total protein is present in a human serum albumin matrix.

The enzyme reference composition of the instant invention also comprises from 60 to 80 weight percent water. Preferably, water comprises from 66 to 70 weight percent of the enzyme reference composition.

The water can be part of the human serum albumin material and/or can be added as a separate constituent.

The pH of enzyme reference composition of the instant is not critical. The optimum pH of the enzyme reference composition will vary depending upon the particular enzymes present therein. In general, the pH can be from 5.0 to 8.7. Preferably, the pH can be from 6 to 8.5. More preferably, the pH can be from 6 to 7. Usually, the preferred pH will be from 6.4 to 6.6. However, ACP has an optional pH of about 6.0.

The pH can be adjusted by any conventional means employed by those skilled in the art, e.g., by the addition of hydrochloric acid (HCl) or sodium hydroxide (NaOH) to the composition.

The enzyme reference composition of the instant invention can also further comprise metabolites, electrolytes, and hormones of known value. Preferably, the enzyme reference composition of the instant invention will further comprise sodium, potassium, calcium, phosphorous, magnesium and chloride in amounts of interest to the clinical chemist.

The enzyme reference composition of the instant invention can be stored in any suitable container, such as a glass ampule.

In general, the enzyme composition of the instant invention can be prepared via the following procedure. A solution having a desired percentage of albumin therein is made by dissolving an appropriate amount of albumin in distilled or de-ionized water. To this albumin solution is then added a sufficient amount of at least one alkylene polyol having 2 to 5 carbons so that the alkylene polyol-albumin solution comprises from 20 to 40 weight percent alkylene polyol, from 60 to 80 weight percent water, and from 3 to 8 gms/dl total protein in a human serum albumin matrix. The pH of the alkylene polyol-albumin solution is then adjusted to a desirable level. After adjusting the pH, the alkylene polyol-albumin solution is assayed for enzyme activity and salt content to obtain baseline values for each constituent of each of the solution's desired constituents. An amount of each of the constituents is then added to the solution so that the enzymes and salts are present in their desired amount.

3

If necessary, the pH of the solution is then readjusted to its optimum level with respect to the enzymes present therein.

The stable enzyme reference composition of the instant invention can be employed as an enzyme reference standard or as an enzyme reference control, i.e., the composition can be employed to either calibrate an instrument or can be employed to periodically verify that the instrument is still operating within the tolerances desired. For the above uses, the enzyme reference composition of the instant invention can contain at least one enzyme in amounts typical of those of interest to those in the clinical laboratory.

The following examples are provided for the purpose of further illustration only and are not intended to be limitations of the disclosed invention.

Example 1

Table III sets forth one preferred embodiment of the enzyme reference composition of the instant invention wherein the enzyme activity is measured at 37°C.

TABLE III

| Test | Specification |
|---|---|
| ACP | 0—20 IU/l |
| ALP | 0—350 IU/l |
| Amylase | 0—400 IU/l |
| CK | 0—600 IU/l |
| SGOT | 0—200 IU/l |
| SGPT | 0—200 IU/l |
| $\gamma$-GT | 0—300 IU/l |
| LDH | 0—500 IU/l |
| Sodium | 125—157 mEq/l |
| Potassium | 3.5—4.5 mEq/l |
| Calcium | 8.4—10.6 mg/dl |
| Phosphorous | 1.6—2.4 mg/dl |
| Magnesium | 1.6—2.4 mg/dl |
| Chloride | 88—112 mEq/l |
| pH | 6.5±0.1 |
| HB$_s$Ag-B* | Negative |
| Total protein (human albumin) | 4.5±0.5 gm/dl |
| Ethylene glycol | 33-1/3% by weight |
| Water | 66-2/3% by weight |

*Hb$_s$Ag-B denotes Hapatitis-surface Antigen Type B

Example 2

Preparation of albumin base enzyme reference composition

Add albumin (Human cohn fraction V) slowly to a 33-1/3% by weight aqueous solution of ethylene glycol with continuous mixing. The albumin is preferably dissolved at room temperature, but extended mixing at 2 to 8°C is acceptable. The amount of albumin added is such that the total protein content is about 4.5±0.5 gm/dl.

The pH of the resulting solution is adjusted, if necessary, to 6.5±0.1 with 6 N HCl or 6 N NaOH.

Next, filter the solution through an ethylene glycol impervious filter material having a final filter porosity of 0.4—0.6$\mu$.

Assay the filtered solution for enzyme activity and salt content to obtain baseline values for each constituent of the composition. Add an amount of each constituent so that the enzymes and salts are present in amounts within the levels set forth in Table III, supra.

If necessary readjust the pH of the enzyme reference composition to 6.5±0.1 with 6 N HCl or 6 N NaOH.

Example 3

One lot of enzyme reference composition within the scope of the instant invention was formulated according to the procedure set forth in Example 2, supra, and stored in plastic vials. These compositions were incubated at various temperatures (—15°, 25°, 32°, 37°, and 41°C) and assayed at 37°C at designated time intervals. The data obtained are set forth in Tables IV—X.

## TABLE IV
### ALP

| Hour | 41°C | | 37°C | | 32°C | | 25°C | | −15°C |
| --- | --- | --- | --- | --- | --- | --- | --- | --- | --- |
| | A | A/E×100% | B | B/E×100% | C | C/E×100% | D | D/E×100% | E |
| 0.0 | 445.0 | 100 | 445.0 | 100 | 445.0 | 100 | 445.0 | 100 | 445.0 |
| 4.0 | 462.0 | 104 | 449.0 | 101 | 455.0 | 102 | 448.0 | 101 | 445.0 |
| 8.0 | 471.0 | 100 | 474.0 | 101 | 479.0 | 102 | 464.0 | 99 | 470.0 |
| 24.0 | 433.0 | 94 | 445.0 | 96 | 460.0 | 100 | 483.0 | 105 | 462.0 |
| 48.0 | 415.0 | 89 | 426.0 | 92 | 448.0 | 97 | 463.0 | 100 | 464.0 |
| 72.0 | 400.0 | 86 | 432.0 | 93 | 444.0 | 95 | 463.0 | 99 | 466.0 |
| 96.0 | 382.0 | 84 | 415.0 | 91 | 435.0 | 95 | 460.0 | 101 | 457.0 |
| 120.0 | 391.0 | 85 | 431.0 | 94 | 445.0 | 97 | 454.0 | 99 | 460.0 |
| 144.0 | 372.0 | 82 | 409.0 | 90 | 420.0 | 93 | 453.0 | 100 | 454.0 |
| 312.0 | 322.0 | 69 | 375.0 | 80 | 415.0 | 88 | 444.0 | 95 | 469.0 |
| 480.0 | 281.0 | 60 | 353.0 | 75 | 398.0 | 85 | 426.0 | 90 | 471.0 |
| 648.0 | 264.0 | 58 | 326.0 | 72 | 378.0 | 84 | 417.0 | 92 | 452.0 |

## TABLE V
### Amylase

| Hour | 41°C | | 37°C | | 32°C | | 25°C | | −15°C |
| --- | --- | --- | --- | --- | --- | --- | --- | --- | --- |
| | A | A/E×100% | B | B/E×100% | C | C/E×100% | D | D/E×100% | E |
| 0.0 | 420.0 | 100 | 420.0 | 100 | 420.0 | 100 | 420.0 | 100 | 420.0 |
| 4.0 | 412.0 | 98 | 405.0 | 96 | 402.0 | 96 | 412.0 | 98 | 420.0 |
| 8.0 | 380.0 | 95 | 397.0 | 100 | 397.0 | 100 | 382.0 | 96 | 398.0 |
| 24.0 | 361.0 | 95 | 368.0 | 96 | 368.0 | 96 | 381.0 | 100 | 382.0 |
| 48.0 | 362.0 | 91 | 372.0 | 94 | 381.0 | 96 | 379.0 | 95 | 397.0 |
| 72.0 | 354.0 | 89 | 373.0 | 94 | 391.0 | 98 | —N/A— | | 397.0 |
| 96.0 | 319.0 | 86 | 357.0 | 96 | 369.0 | 99 | —N/A— | | 371.0 |
| 120.0 | 329.0 | 84 | —N/A[1]— | | 377.0 | 96 | 386.0 | 99 | 391.0 |
| 312.0 | 295.0 | 75 | 377.0 | 96 | 389.0 | 99 | 394.0 | 101 | 392.0 |
| 480.0 | 291.0 | 74 | 375.0 | 95 | 407.0 | 103 | 382.0 | 97 | 394.0 |
| 648.0 | 306.0 | 75 | 381.0 | 94 | 408.0 | 100 | 414.0 | 102 | 406.0 |

[1] N/A denotes not available.

O 016 573

## TABLE VI
### CK

| Hour | 41°C A | A/Ex100% | 37°C B | B/Ex100% | 32°C C | C/Ex100% | 25°C D | D/Ex100% | −15°C E |
|---|---|---|---|---|---|---|---|---|---|
| 0.0 | 559.0 | 100 | 559.0 | 100 | 559.0 | 100 | 599.0 | 100 | 559.0 |
| 4.0 | 543.0 | 97 | 551.0 | 99 | 558.0 | 100 | 533.0 | 99 | 559.0 |
| 8.0 | 515.0 | 93 | 544.0 | 98 | 550.0 | 99 | 559.0 | 101 | 554.0 |
| 24.0 | 417.0 | 75 | 514.0 | 92 | 544.0 | 98 | 549.0 | 99 | 557.0 |
| 48.0 | 300.0 | 54 | 474.0 | 86 | 529.0 | 95 | 554.0 | 100 | 554.0 |
| 72.0 | 207.0 | 38 | 450.0 | 83 | 517.0 | 95 | 524.0 | 97 | 543.0 |
| 96.0 | —N/A[1]— | | 410.0 | 76 | 499.0 | 93 | 534.0 | 99 | 539.0 |
| 120.0 | —N/A— | | 408.0 | 73 | 519.0 | 92 | 527.0 | 94 | 562.0 |
| 144.0 | —N/A— | | 377.0 | 68 | 507.0 | 92 | 552.0 | 100 | 551.0 |
| 312.0 | —N/A— | | 259.0 | 46 | 476.0 | 85 | 543.0 | 97 | 559.0 |
| 480.0 | —N/A— | | —N/A— | | 445.0 | 80 | 538.0 | 96 | 558.0 |
| 648.0 | —N/A— | | —N/A— | | 415.0 | 74 | 503.0 | 90 | 558.0 |

[1] N/A denotes not available

## TABLE VII
### SGOT

| Hour | 41°C A | A/Ex100% | 37°C B | B/Ex100% | 32°C C | C/Ex100% | 25°C D | D/Ex100% | −15°C E |
|---|---|---|---|---|---|---|---|---|---|
| 0.0 | 464.0 | 100 | 464.0 | 100 | 464.0 | 100 | 464.0 | 100 | 464.0 |
| 4.0 | 463.0 | 100 | 458.0 | 99 | 467.0 | 101 | 460.0 | 99 | 464.0 |
| 8.0 | 454.0 | 99 | 459.0 | 100 | 458.0 | 100 | 462.0 | 100 | 460.0 |
| 24.0 | 439.0 | 96 | 449.0 | 98 | 454.0 | 99 | 453.0 | 99 | 457.0 |
| 48.0 | 421.0 | 92 | 435.0 | 95 | 449.0 | 98 | 459.0 | 100 | 460.0 |
| 72.0 | 418.0 | 88 | 456.0 | 96 | 466.0 | 98 | 467.0 | 99 | 474.0 |
| 96.0 | 389.0 | 82 | 428.0 | 90 | 451.0 | 95 | 472.0 | 100 | 474.0 |
| 120.0 | 368.0 | 79 | 413.0 | 89 | 439.0 | 94 | 448.0 | 96 | 466.0 |
| 144.0 | 347.0 | 75 | 395.0 | 85 | 426.0 | 92 | 455.0 | 98 | 462.0 |
| 312.0 | 238.0 | 51 | 321.0 | 69 | 388.0 | 83 | 439.0 | 94 | 466.0 |
| 480.0 | 153.0 | 33 | 254.0 | 55 | 346.0 | 74 | 423.0 | 91 | 465.0 |
| 648.0 | —N/A[1]— | | 290.0 | 45 | 313.0 | 68 | 408.0 | 88 | 462.0 |

[1] N/A denotes not available.

## TABLE VIII
### SGPT

| Hour | 41°C | | 37°C | | 32°C | | 25°C | | −15°C |
|---|---|---|---|---|---|---|---|---|---|
| | A | A/E×100% | B | B/E×100% | C | C/E×100% | D | D/E×100% | E |
| 0.0 | 522.0 | 100 | 522.0 | 100 | 522.0 | 100 | 522.0 | 100 | 522.0 |
| 4.0 | 509.0 | 98 | 517.0 | 99 | 517.0 | 99 | 519.0 | 99 | 522.0 |
| 8.0 | 473.0 | 91 | 478.0 | 92 | 493.0 | 95 | 511.0 | 98 | 521.0 |
| 24.0 | 438.0 | 84 | 475.0 | 91 | 488.0 | 94 | 511.0 | 98 | 521.0 |
| 48.0 | 418.0 | 78 | 458.0 | 86 | 488.0 | 92 | 514.0 | 96 | 533.0 |
| 72.0 | 369.0 | 74 | 418.0 | 84 | 466.0 | 94 | —N/A[1]— | | 496.0 |
| 96.0 | 357.0 | 68 | 418.0 | 80 | 474.0 | 90 | 508.0 | 97 | 525.0 |
| 120.0 | 329.0 | 66 | 411.0 | 83 | 453.0 | 91 | 487.0 | 98 | 497.0 |

[1] N/A denotes not available.

## TABLE IX
### γ-GT

| Hour | 41°C | | 37°C | | 32°C | | 25°C | | −15°C |
|---|---|---|---|---|---|---|---|---|---|
| | A | A/E×100% | B | B/E×100% | C | C/E×100% | D | D/E×100% | E |
| 0.0 | 139.0 | 100 | 139.0 | 100 | 139.0 | 100 | 139.0 | 100 | 139.0 |
| 4.0 | 139.0 | 100 | 136.0 | 98 | 139.0 | 100 | 143.0 | 103 | 139.0 |
| 8.0 | 138.0 | 101 | 140.0 | 102 | 137.0 | 100 | 139.0 | 101 | 137.0 |
| 24.0 | 133.0 | 96 | 137.0 | 99 | 133.0 | 96 | 139.0 | 101 | 138.0 |
| 48.0 | 135.0 | 98 | 136.0 | 99 | 138.0 | 100 | 138.0 | 100 | 138.0 |
| 72.0 | 135.0 | 96 | 143.0 | 101 | 143.0 | 101 | 140.0 | 99 | 141.0 |
| 96.0 | 136.0 | 97 | 142.0 | 101 | 142.0 | 101 | 142.0 | 101 | 140.0 |
| 120.0 | 142.0 | 96 | 148.0 | 100 | 146.0 | 99 | 143.0 | 97 | 148.0 |
| 144.0 | 137.0 | 98 | 141.0 | 101 | 139.0 | 99 | 144.0 | 103 | 140.0 |
| 312.0 | 129.0 | 91 | 135.0 | 96 | 139.0 | 99 | 141.0 | 100 | 141.0 |
| 480.0 | 124.0 | 91 | 133.0 | 97 | 136.0 | 99 | 137.0 | 100 | 137.0 |
| 648.0 | 121.0 | 89 | 132.0 | 97 | 136.0 | 100 | 136.0 | 100 | 136.0 |

0 016 573

TABLE X
LDH

| | 41°C | | 37°C | | 32°C | | 25°C | | −15°C |
|---|---|---|---|---|---|---|---|---|---|
| Hour | A | A/E×100% | B | B/E×100% | C | C/E×100% | D | D/E×100% | E |
| 0.0 | 662.0 | 100 | 662.0 | 100 | 662.0 | 100 | 662.0 | 100 | 662.0 |
| 4.0 | 652.0 | 98 | 655.0 | 99 | 651.0 | 98 | 651.0 | 98 | 662.0 |
| 8.0 | 617.0 | 97 | 623.0 | 98 | 626.0 | 99 | 633.0 | 100 | 633.0 |
| 24.0 | 591.0 | 94 | 603.0 | 96 | 609.0 | 97 | 622.0 | 99 | 627.0 |
| 48.0 | 585.0 | 94 | 604.0 | 97 | 609.0 | 98 | 608.0 | 98 | 621.0 |
| 72.0 | 555.0 | 92 | 556.0 | 93 | 584.0 | 97 | 590.0 | 98 | 601.0 |
| 96.0 | 563.0 | 91 | 585.0 | 95 | 597.0 | 96 | 612.0 | 99 | 619.0 |
| 120.0 | 553.0 | 89 | 572.0 | 92 | 593.0 | 96 | 601.0 | 97 | 619.0 |

Table XI sets forth an Arrhenius plot for 90% life based upon the data set forth in Tables IV—X.

TABLE XI

Arrhenius plot for 90% life based on temperatures 41°, 37°, 32°, and 25°C

| Temperature | ALP | Amylase | CK | SGOT | SGPT | $\gamma$-GT | LDH |
|---|---|---|---|---|---|---|---|
| 4°C | 9.9 M[1] | 6.8 Y | 145.9 Y | 2.4 Y | 6.6 Y | 206.9 Y | 1.0 Y |
| −15°C | 10.2 Y[2] | 132.1 Y | 208452.5 Y | 109.8 Y | 734.9 Y | N/A[3] | 14.8 Y |

[1] M denotes months
[2] Y denotes years
[3] N/A denotes not available.

The data in Tables IV—X as well as Table XI's Arrhenius plot based thereon demonstrate that the enzyme reference compositions within the scope of this invention are stable for periods of time far exceeding the shelf life of enzyme constituents present in prior art reference compositions.

The following examples are provided for the purpose of distinguishing the instant invention over the invention set forth in Maurukas I and Maurakas II, supra.

Example 4

Lots of enzyme reference compositions within the scope of the instant invention were formulated according to the procedure set forth in Example 2, supra, save that when the pH of a particular lot was other than 6.5±0.1, that lot's pH was adjusted accordingly with either 6N HCl or 6N NaOH. These lots were stored in plastic vials, incubated at various temperatures, and assayed at 37°C at designated time intervals. The data obtained are set forth in Tables XII—XXI.

TABLE XII
ALP @ pH 6.5 in Human serum albumin matrix

| Hour | 41°C | | 37°C | | 32°C | | 25°C | | −15°C |
| | A | A/E×100% | B | B/E×100% | C | C/E×100% | D | D/E×100% | E |
|---|---|---|---|---|---|---|---|---|---|
| 0.0 | 107.0 | 100 | 107.0 | 100 | 107.0 | 100 | 107.0 | 100 | 107.0 |
| 4.0 | 110.0 | 103 | 167.0 | 156 | 109.0 | 102 | 108.0 | 101 | 107.0 |
| 8.0 | 113.0 | 97 | 115.0 | 99 | 115.0 | 99 | 111.0 | 96 | 116.0 |
| 24.0 | 103.0 | 96 | 106.0 | 99 | 109.0 | 102 | 107.0 | 100 | 107.0 |
| 48.0 | 101.0 | 89 | 106.0 | 93 | 107.0 | 94 | 110.0 | 96 | 114.0 |
| 72.0 | 98.0 | 89 | 103.0 | 94 | 108.0 | 98 | 112.0 | 102 | 110.0 |
| 96.0 | 94.0 | 84 | 100.0 | 89 | 104.0 | 93 | 110.0 | 98 | 112.0 |
| 120.0 | 95.0 | 81 | 104.0 | 89 | 105.0 | 90 | 106.0 | 91 | 117.0 |
| 144.0 | 88.0 | 78 | 99.0 | 88 | 102.0 | 90 | 109.0 | 96 | 113.0 |
| 312.0 | 78.0 | 68 | 94.0 | 82 | 100.0 | 88 | 108.0 | 95 | 114.0 |
| 480.0 | 70.0 | 64 | 85.0 | 77 | 94.0 | 85 | 103.0 | 94 | 110.0 |
| 648.0 | 66.0 | 60 | 77.0 | 70 | 89.0 | 81 | 97.0 | 88 | 110.0 |

TABLE XIII
ALP @ pH 8.5 in Human serum albumin matrix

| Hour | 41°C | | 37°C | | 32°C | | −15°C |
| | A | A/D×100% | B | B/D×100% | C | C/D×100% | D |
|---|---|---|---|---|---|---|---|
| 0.0 | 686.0 | 100 | 686.0 | 100 | 686.0 | 100 | 686.0 |
| 4.0 | 719.0 | 105 | 672.0 | 98 | 679.0 | 99 | 686.0 |
| 8.0 | 744.0 | 99 | 727.0 | 96 | 826.0 | 109 | 755.0 |
| 72.0 | 740.0 | 98 | 787.0 | 104 | 788.0 | 104 | 755.0 |
| 96.0 | 754.0 | 97 | 822.0 | 106 | 801.0 | 103 | 744.0 |
| 168.0 | —N/A[1]— | | 744.0 | 99 | —N/A— | | 748.0 |

[1] N/A denotes not available.

## TABLE XIV
### CK @ pH 6.5 in Human serum albumin matrix

| Hour | 41°C | | 37°C | | 32°C | | 25°C | | −15°C |
| --- | --- | --- | --- | --- | --- | --- | --- | --- | --- |
| | A | A/E×100% | B | B/E×100% | C | C/E×100% | D | D/E×100% | E |
| 0.0 | 340.0 | 100 | 340.0 | 100 | 340.0 | 100 | 340.0 | 100 | 340.0 |
| 4.0 | 330.0 | 97 | 325.0 | 96 | 335.0 | 99 | 332.0 | 98 | 340.0 |
| 8.0 | 309.9 | 93 | 310.0 | 93 | 335.0 | 101 | 337.0 | 101 | 333.0 |
| 24.0 | 249.0 | 75 | 305.0 | 91 | 322.0 | 96 | 331.0 | 99 | 334.0 |
| 48.0 | 184.0 | 55 | 285.0 | 85 | 316.0 | 94 | 330.0 | 99 | 335.0 |
| 72.0 | 130.0 | 39 | 268.0 | 80 | 312.0 | 94 | 321.0 | 96 | 333.0 |
| 96.0 | —N/A[1]— | | 248.0 | 76 | 298.0 | 92 | 319.0 | 98 | 325.0 |
| 120.0 | —N/A— | | 245.0 | 72 | 312.0 | 91 | 324.0 | 95 | 342.0 |
| 144.0 | —N/A— | | 231.0 | 70 | 303.0 | 91 | 328.0 | 99 | 332.0 |

[1] N/A denotes not available.

## TABLE XV
### CK @ pH 8.5 in Human serum albumin matrix

| Hour | 41°C | | 37°C | | 32°C | | −15°C |
| --- | --- | --- | --- | --- | --- | --- | --- |
| | A | A/D×100% | B | B/D×100% | C | C/D×100% | D |
| 0.0 | 464.0 | 100 | 464.0 | 100 | 464.0 | 100 | 464.0 |
| 4.0 | 440.0 | 95 | 481.0 | 104 | 451.0 | 97 | 464.0 |
| 8.0 | 315.0 | 65 | 425.0 | 88 | 507.0 | 105 | 485.0 |
| 24.0 | 62.0 | 13 | 303.0 | 65 | 415.0 | 89 | 466.0 |
| 48.0 | —N/A[1]— | | 209.0 | 43 | 393.0 | 81 | 483.0 |
| 72.0 | —N/A— | | —N/A— | | —N/A— | | 485.0 |
| 96.0 | —N/A— | | —N/A— | | 317.0 | 70 | 450.0 |
| 168.0 | —N/A— | | —N/A— | | 445.0 | 92 | 482.0 |
| 336.0 | —N/A— | | —N/A— | | 201.0 | 43 | 465.0 |

[1] N/A denotes not available.

### TABLE XVI
### SGOT @ pH 6.0 in Human serum albumin matrix

| Hour | 41°C | | 37°C | | 32°C | | 25°C | | −15°C |
|---|---|---|---|---|---|---|---|---|---|
| | A | A/E×100% | B | B/E×100% | C | C/E×100% | D | D/E×100% | E |
| 0.0 | 194.0 | 100 | 194.0 | 100 | 194.0 | 100 | 194.0 | 100 | 194.0 |
| 24.0 | 187.0 | 96 | 189.0 | 97 | 191.0 | 98 | 192.0 | 99 | 194.0 |
| 48.0 | 179.0 | 92 | 186.0 | 96 | 191.0 | 98 | 190.0 | 98 | 194.0 |
| 168.0 | 153.0 | 79 | 169.0 | 87 | 178.0 | 92 | 186.0 | 96 | 194.0 |

### TABLE XVII
### SGOT @ pH 8.5 in Human serum albumin matrix

| Hour | 41°C | | 37°C | | 32°C | | −15°C |
|---|---|---|---|---|---|---|---|
| | A | A/D×100% | B | B/D×100% | C | C/D×100% | D |
| 0.0 | 288.0 | 100 | 288.0 | 100 | 288.0 | 100 | 288.0 |
| 4.0 | 289.0 | 100 | 279.0 | 97 | 276.0 | 96 | 288.0 |
| 8.0 | 273.0 | 98 | 272.0 | 98 | 286.0 | 103 | 278.0 |
| 24.0 | 301.0 | 99 | 304.0 | 100 | 298.0 | 98 | 305.0 |
| 48.0 | 286.0 | 99 | 288.0 | 99 | 244.0 | 84 | 290.0 |
| 72.0 | 291.0 | 98 | 290.0 | 97 | 293.0 | 98 | 298.0 |
| 96.0 | 294.0 | 98 | 295.0 | 99 | 294.0 | 98 | 299.0 |
| 168.0 | —N/A[1]— | | 300.0 | 99 | 246.0 | 81 | 304.0 |
| 336.0 | —N/A— | | 271.0 | 93 | 280.0 | 96 | 292.0 |

[1] N/A denotes not available.

### TABLE XVIII
### SGPT @ pH 6.0 in Human serum albumin matrix

| Hour | 41°C | | 37°C | | 32°C | | 25°C | | −15°C |
|---|---|---|---|---|---|---|---|---|---|
| | A | A/E×100% | B | B/E×100% | C | C/E×100% | D | D/E×100% | E |
| 0.0 | 30.0 | 100 | 30.0 | 100 | 30.0 | 100 | 30.0 | 100 | 30.0 |
| 8.0 | 28.0 | 93 | 30.0 | 100 | 31.0 | 103 | 32.0 | 107 | 30.0 |
| 24.0 | 29.0 | 97 | 32.0 | 107 | 30.0 | 100 | 31.0 | 103 | 30.0 |
| 48.0 | 26.0 | 87 | 28.0 | 93 | 30.0 | 100 | 31.0 | 103 | 30.0 |
| 168.0 | 22.0 | 73 | 25.0 | 83 | 26.0 | 87 | 29.0 | 97 | 30.0 |

TABLE XIX
SGPT @ pH 8.5 in Human serum albumin matrix

| | 41°C | | 37°C | | 32°C | | −15°C |
|---|---|---|---|---|---|---|---|
| Hour | A | A/D×100% | B | B/D×100% | C | C/D×100% | D |
| 0.0 | 255.0 | 100 | 255.0 | 100 | 255.0 | 100 | 255.0 |
| 4.0 | 250.0 | 98 | 239.0 | 94 | 246.0 | 96 | 255.0 |
| 8.0 | 218.0 | 94 | 216.0 | 94 | 246.0 | 106 | 231.0 |
| 24.0 | 191.0 | 79 | 215.0 | 89 | 222.0 | 92 | 241.0 |
| 48.0 | 150.0 | 70 | 193.0 | 91 | 218.0 | 102 | 213.0 |
| 72.0 | 140.0 | 56 | 201.0 | 80 | 227.0 | 91 | 250.0 |
| 96.0 | 107.0 | 46 | 175.0 | 75 | 206.0 | 88 | 234.0 |
| 168.0 | —N/A[1]— | | 140.0 | 63 | 209.0 | 95 | 221.0 |
| 336.0 | —N/A— | | 29.0 | 14 | 75.0 | 35 | 213.0 |

[1] N/A denotes not available.

TABLE XX
LDH @ pH 6.5 in Human serum albumin matrix

| | 41°C | | 37°C | | 32°C | | −15°C |
|---|---|---|---|---|---|---|---|
| Hour | A | A/D×100% | B | B/D×100% | C | C/D×100% | D |
| 0.0 | 416.0 | 100 | 416.0 | 100 | 416.0 | 100 | 416.0 |
| 4.0 | 402.0 | 97 | 407.0 | 98 | 410.0 | 99 | 416.0 |
| 8.0 | 407.0 | 104 | 408.0 | 104 | 410.0 | 105 | 392.0 |
| 24.0 | 447.0 | 112 | 492.0 | 123 | 483.0 | 121 | 400.0 |
| 48.0 | 378.0 | 92 | 394.0 | 96 | 394.0 | 96 | 411.0 |
| 72.0 | 420.0 | 91 | 432.0 | 94 | 460.0 | 100 | 461.0 |
| 96.0 | 346.0 | 85 | 378.0 | 93 | 396.0 | 98 | 406.0 |
| 168.0 | 381.0 | 85 | —N/A[1]— | | 417.0 | 93 | 447.0 |
| 336.0 | 343.0 | 83 | 370.0 | 90 | 377.0 | 92 | 411.0 |
| 505.0 | 360.0 | 84 | 372.0 | 87 | 394.0 | 92 | 427.0 |

[1] N/A denotes not available.

TABLE XXI
LDH @ pH 8.5 in Human serum albumin matrix

| | 41°C | | 37°C | | 32°C | | −15°C |
|---|---|---|---|---|---|---|---|
| Hour | A | A/D×100% | B | B/D×100% | C | C/D×100% | D |
| 0.0 | 402.0 | 100 | 402.0 | 100 | 402.0 | 100 | 402.0 |
| 4.0 | 395.0 | 98 | 393.0 | 98 | 397.0 | 99 | 402.0 |
| 8.0 | 380.0 | 96 | 385.0 | 97 | 402.0 | 101 | 397.0 |
| 24.0 | 425.0 | 108 | 442.0 | 113 | 456.0 | 116 | 392.0 |
| 48.0 | 359.0 | 89 | 375.0 | 93 | 382.0 | 95 | 403.0 |
| 72.0 | 400.0 | 89 | 422.0 | 94 | 434.0 | 96 | 450.0 |
| 96.0 | 338.0 | 87 | 359.0 | 93 | 374.0 | 97 | 387.0 |
| 168.0 | 380.0 | 85 | 389.0 | 87 | 417.0 | 93 | 448.0 |
| 336.0 | —N/A[1]— | | 347.0 | 88 | 360.0 | 91 | 395.0 |

[1] N/A denotes not available.

Example 5

Lots of enzyme reference compositions of the type described by Maurukas I and Maurukas II, supra, were formulated according to the improved procedure set forth in U.S.—A—4,158,544. These lots were stored in plastic vials, incubated at various temperatures, and assayed at 37°C at designated time intervals. The data obtained are set forth in Tables XXII—XXXI.

TABLE XXII
ALP @ pH 6.5 in Human serum matrix

| Hour | 41°C A | A/D×100% | 37°C B | B/D×100% | 32°C C | C/D×100% | −15°C D |
|---|---|---|---|---|---|---|---|
| 0.0 | 51.0 | 100 | 51.0 | 100 | 51.0 | 100 | 51.0 |
| 4.0 | 54.0 | 106 | 54.0 | 106 | 54.0 | 106 | 51.0 |
| 8.0 | 57.0 | 112 | 56.0 | 110 | 55.0 | 108 | 51.0 |
| 24.0 | 50.0 | 94 | 54.0 | 102 | 57.0 | 108 | 53.0 |
| 48.0 | 47.0 | 85 | 51.0 | 93 | 57.0 | 104 | 55.0 |
| 72.0 | 37.0 | 70 | 46.0 | 87 | 52.0 | 98 | 53.0 |
| 96.0 | 34.0 | 64 | 43.0 | 81 | 50.0 | 94 | 53.0 |
| 121.0 | 33.0 | 60 | 43.0 | 78 | 51.0 | 93 | 55.0 |
| 144.0 | 30.0 | 55 | 42.0 | 76 | 48.0 | 87 | 55.0 |
| 336.0 | 18.0 | 35 | 31.0 | 60 | 39.0 | 75 | 52.0 |
| 504.0 | —N/A[1]— | | 32.0 | 55 | 43.0 | 74 | 58.0 |
| 1344.0 | —N/A— | | 24.0 | 35 | 43.0 | 62 | 69.0 |
| 2016.0 | —N/A— | | —N/A— | | 29.0 | 51 | 57.0 |

[1] N/A denotes not available.

TABLE XXIII
ALP @ pH 8.5 in Human serum matrix

| Hour | 41°C A | A/D×100% | 37°C B | B/D×100% | 32°C C | C/D×100% | −15°C D |
|---|---|---|---|---|---|---|---|
| 0.0 | 295.0 | 100 | 295.0 | 100 | 295.0 | 100 | 295.0 |
| 4.0 | 298.0 | 101 | 295.0 | 100 | 294.0 | 100 | 295.0 |
| 8.0 | 288.0 | 99 | 309.0 | 106 | 309.0 | 106 | 291.0 |
| 24.0 | 263.0 | 89 | —N/A[1]— | | 292.0 | 99 | 294.0 |
| 48.0 | 235.0 | 76 | 283.0 | 92 | 292.0 | 94 | 309.0 |
| 72.0 | 223.0 | 77 | 295.0 | 102 | 312.0 | 108 | 289.0 |
| 96.0 | 187.0 | 64 | 274.0 | 94 | 302.0 | 103 | 293.0 |
| 172.0 | 114.0 | 39 | 220.0 | 75 | 258.0 | 88 | 293.0 |

[1] N/A denotes not available.

TABLE XXIV
CK @ pH 6.5 in Human serum matrix

| Hour | 41°C A | A/D×100% | 37°C B | B/D×100% | 32°C C | C/D×100% | −15°C D |
|---|---|---|---|---|---|---|---|
| 0.0 | 278.0 | 100 | 278.0 | 100 | 278.0 | 100 | 278.0 |
| 4.0 | 286.0 | 103 | 285.0 | 103 | 281.0 | 101 | 278.0 |
| 8.0 | 260.0 | 87 | 261.0 | 87 | 263.0 | 88 | 300.0 |
| 24.0 | 210.0 | 73 | 261.0 | 90 | 272.0 | 94 | 289.0 |
| 48.0 | 156.0 | 53 | 252.0 | 86 | 269.0 | 92 | 293.0 |
| 72.0 | —N/A[1]— | | 238.0 | 83 | 260.0 | 90 | 288.0 |
| 96.0 | —N/A— | | 213.0 | 73 | 247.0 | 85 | 292.0 |
| 121.0 | —N/A— | | 195.0 | 66 | 241.0 | 82 | 294.0 |
| 144.0 | —N/A— | | 183.0 | 62 | 236.0 | 80 | 294.0 |
| 336.0 | —N/A— | | 101.0 | 40 | 167.0 | 65 | 255.0 |
| 504.0 | —N/A— | | —N/A— | | 157.0 | 58 | 270.0 |

[1] N/A denotes not available.

14

### TABLE XXV
#### CK @ pH 8.5 in Human serum matrix

| Hour | 41°C | | 37°C | | 32°C | | −15°C |
|---|---|---|---|---|---|---|---|
| | A | A/D×100% | B | B/D×100% | C | C/D×100% | D |
| 0.0 | 127.0 | 100 | 127.0 | 100 | 127.0 | 100 | 127.0 |
| 4.0 | 115.0 | 91 | 129.0 | 102 | 124.0 | 98 | 127.0 |
| 8.0 | 71.0 | 55 | 119.0 | 93 | 132.0 | 103 | 128.0 |
| 24.0 | 2.0 | 2 | —N/A[1]— | | 99.0 | 85 | 117.0 |
| 48.0 | —N/A— | | 28.0 | 24 | 83.0 | 71 | 117.0 |
| 72.0 | —N/A— | | —N/A— | | 68.0 | 56 | 122.0 |
| 96.0 | —N/A— | | —N/A— | | 56.0 | 43 | 131.0 |

[1] N/A denotes not available.

### TABLE XXVI
#### SGOT @ pH 6.0 in Human serum matrix

| Hour | 41°C | | 25°C | | 4°C | | −15°C |
|---|---|---|---|---|---|---|---|
| | A | A/D×100% | B | B/D×100% | C | C/D×100% | D |
| 0.0 | 111.0 | 100 | 111.0 | 100 | 111.0 | 100 | 111.0 |
| 1.0 | 110.0 | 99 | —N/A[1]— | | —N/A— | | 111.0 |
| 2.0 | 111.0 | 100 | —N/A— | | —N/A— | | 111.0 |
| 3.0 | 109.0 | 98 | —N/A— | | —N/A— | | 111.0 |
| 4.0 | 107.0 | 96 | 112.0 | 101 | 111.0 | 100 | 111.0 |
| 5.0 | 121.0 | 102 | —N/A— | | —N/A— | | 118.0 |
| 6.0 | 114.0 | 96 | —N/A— | | —N/A— | | 118.0 |
| 7.0 | 111.0 | 94 | —N/A— | | —N/A— | | 118.0 |
| 8.0 | 106.0 | 90 | —N/A— | | —N/A— | | 118.0 |
| 9.0 | 103.0 | 87 | 121.0 | 102 | 118.0 | 100 | 118.0 |
| 10.0 | 86.0 | 76 | —N/A— | | —N/A— | | 114.0 |
| 11.0 | 87.0 | 77 | —N/A— | | —N/A— | | 114.0 |
| 12.0 | 91.0 | 80 | —N/A— | | —N/A— | | 114.0 |
| 15.0 | 79.0 | 70 | 108.0 | 96 | 112.0 | 99 | 113.0 |
| 24.0 | 49.0 | 43 | 108.0 | 95 | 113.0 | 100 | 114.0 |
| 30.0 | 43.0 | 38 | —N/A— | | —N/A— | | 113.0 |
| 36.0 | 36.0 | 32 | 103.0 | 91 | 112.0 | 99 | 113.0 |
| 48.0 | 22.0 | 20 | 98.0 | 88 | 111.0 | 99 | 112.0 |
| 56.0 | 17.0 | 15 | 98.0 | 87 | 112.0 | 99 | 113.0 |
| 72.0 | <5.0 | N/A | 95.0 | 84 | 112.0 | 99 | 114.0 |
| 80.0 | <5.0 | N/A | 93.0 | 83 | 112.0 | 100 | 113.0 |

[1] N/A denotes not available.

### TABLE XXVII
### SGOT @ pH 8.5 in Human serum matrix

| | 41°C | | 25°C | | 4°C | | −15°C |
|---|---|---|---|---|---|---|---|
| Hour | A | A/D×100% | B | B/D×100% | C | C/D×100% | D |
| 0.0 | 124.0 | 100 | 124.0 | 100 | 124.0 | 100 | 124.0 |
| 1.0 | 124.0 | 99 | —N/A[1]— | | —N/A— | | 124.0 |
| 2.0 | 125.0 | 101 | —N/A— | | —N/A— | | 124.0 |
| 3.0 | 123.0 | 99 | —N/A— | | —N/A— | | 124.0 |
| 4.0 | 123.0 | 99 | 124.0 | 100 | 124.0 | 100 | 124.0 |
| 5.0 | 136.0 | 103 | —N/A— | | —N/A— | | 132.0 |
| 6.0 | 135.0 | 102 | —N/A— | | —N/A— | | 132.0 |
| 7.0 | 132.0 | 100 | —N/A— | | —N/A— | | 132.0 |
| 8.0 | 130.0 | 98 | —N/A— | | —N/A— | | 132.0 |
| 9.0 | 129.0 | 98 | 132.0 | 100 | 130.0 | 98 | 132.0 |
| 10.0 | 117.0 | 92 | —N/A— | | —N/A— | | 127.0 |
| 11.0 | 117.0 | 92 | —N/A— | | —N/A— | | 127.0 |
| 12.0 | 121.0 | 95 | —N/A— | | —N/A— | | 127.0 |
| 15.0 | 122.0 | 96 | 120.0 | 94 | 125.0 | 98 | 128.0 |
| 24.0 | 111.0 | 87 | 126.0 | 99 | 127.0 | 100 | 127.0 |
| 30.0 | 107.0 | 85 | —N/A— | | —N/A— | | 126.0 |
| 36.0 | 101.0 | 80 | 125.0 | 99 | 126.0 | 100 | 126.0 |
| 48.0 | 90.0 | 71 | 124.0 | 98 | 126.0 | 100 | 126.0 |
| 56.0 | 80.0 | 63 | 124.0 | 98 | 126.0 | 100 | 127.0 |
| 72.0 | 64.0 | 50 | 123.0 | 96 | 127.0 | 99 | 128.0 |
| 80.0 | 59.0 | 46 | 123.0 | 96 | 127.0 | 100 | 128.0 |

[1] N/A denotes not available.

### TABLE XXVIII
### SGPT @ pH 6.0 in Human serum matrix

| | 41°C | | 25°C | | 4°C | | −15°C |
|---|---|---|---|---|---|---|---|
| Hour | A | A/D×100% | B | B/D×100% | C | C/D×100% | D |
| 0.0 | 16.0 | 102 | 16.0 | 102 | 16.0 | 102 | 16.0 |
| 1.0 | 18.0 | 115 | —N/A[1]— | | —N/A— | | 16.0 |
| 2.0 | 19.0 | 121 | —N/A— | | —N/A— | | 16.0 |
| 3.0 | 20.0 | 128 | —N/A— | | —N/A— | | 16.0 |
| 4.0 | 20.0 | 100 | 18.0 | 90 | 20.0 | 100 | 20.0 |
| 5.0 | 12.0 | 68 | —N/A— | | —N/A— | | 18.0 |
| 6.0 | 13.0 | 74 | —N/A— | | —N/A— | | 18.0 |
| 7.0 | 14.0 | 80 | —N/A— | | —N/A— | | 18.0 |
| 8.0 | 15.0 | 85 | —N/A— | | —N/A— | | 18.0 |
| 9.0 | 17.0 | 97 | 18.0 | 102 | 17.0 | 97 | 18.0 |
| 10.0 | 22.0 | 90 | —N/A— | | —N/A— | | 24.0 |
| 11.0 | 7.0 | 29 | —N/A— | | —N/A— | | 24.0 |
| 12.0 | 29.0 | 119 | —N/A— | | —N/A— | | 24.0 |
| 15.0 | 15.0 | 88 | 16.0 | 94 | 17.0 | 100 | 17.0 |
| 24.0 | 11.0 | 45 | 29.0 | 119 | 13.0 | 53 | 24.0 |
| 30.0 | 12.0 | 72 | —N/A— | | —N/A— | | 17.0 |
| 36.0 | 10.0 | 60 | 16.0 | 95 | 17.0 | 101 | 17.0 |
| 48.0 | 8.0 | 47 | 16.0 | 94 | 16.0 | 94 | 17.0 |
| 56.0 | 8.0 | 42 | 16.0 | 84 | 17.0 | 89 | 19.0 |
| 72.0 | 7.0 | 41 | 16.0 | 95 | 17.0 | 101 | 17.0 |
| 80.0 | <5.0 | —N/A— | 16.0 | 104 | 17.0 | 111 | 15.0 |

[1] N/A denotes not available.

### TABLE XXIX
SGPT @ pH 8.5 in Human serum matrix

| Hour | 41°C | | 25°C | | 4°C | | −15°C |
| --- | --- | --- | --- | --- | --- | --- | --- |
| | A | A/D×100% | B | B/D×100% | C | C/D×100% | D |
| 0.0 | 20.0 | 100 | 20.0 | 100 | 20.0 | 100 | 20.0 |
| 1.0 | 19.0 | 95 | —N/A[1]— | | —N/A— | | 20.0 |
| 2.0 | 20.0 | 100 | —N/A— | | —N/A— | | 20.0 |
| 3.0 | 20.0 | 100 | —N/A— | | —N/A— | | 20.0 |
| 4.0 | 20.0 | 92 | 22.0 | 101 | 21.0 | 96 | 22.0 |
| 5.0 | 12.0 | 55 | —N/A— | | —N/A— | | 22.0 |
| 6.0 | 13.0 | 60 | —N/A— | | —N/A— | | 22.0 |
| 7.0 | 15.0 | 69 | —N/A— | | —N/A— | | 22.0 |
| 8.0 | 16.0 | 74 | —N/A— | | —N/A— | | 22.0 |
| 9.0 | 18.0 | 83 | 20.0 | 92 | 22.0 | 101 | 22.0 |
| 10.0 | 6.0 | 42 | —N/A— | | —N/A— | | 14.0 |
| 11.0 | <5.0 | N/A | —N/A— | | —N/A— | | 14.0 |
| 12.0 | 13.0 | 91 | —N/A— | | —N/A— | | 14.0 |
| 15.0 | 5.0 | 50 | 10.0 | 100 | 11.0 | 110 | 10.0 |
| 24.0 | 8.0 | 56 | 15.0 | 104 | 15.0 | 104 | 14.0 |
| 30.0 | 9.0 | 50 | —N/A— | | —N/A— | | 18.0 |
| 36.0 | 7.0 | 39 | 17.0 | 94 | 18.0 | 100 | 18.0 |
| 48.0 | <5.0 | N/A | 15.0 | 80 | 17.0 | 91 | 19.0 |
| 56.0 | <5.0 | N/A | 15.0 | 86 | 18.0 | 103 | 17.0 |
| 72.0 | <5.0 | N/A | 18.0 | 93 | 20.0 | 103 | 19.0 |
| 80.0 | <5.0 | N/A | 13.0 | 67 | 19.0 | 98 | 19.0 |

[1] N/A denotes not available.

### TABLE XXX
LDH @ pH 6.5 in Human serum matrix

| Hour | 41°C | | 37°C | | 32°C | | −15°C |
| --- | --- | --- | --- | --- | --- | --- | --- |
| | A | A/D×100% | B | B/D×100% | C | C/D×100% | D |
| 0.0 | 430.0 | 100 | 430.0 | 100 | 430.0 | 100 | 430.0 |
| 4.0 | 420.0 | 98 | 434.0 | 101 | 426.0 | 99 | 430.0 |
| 8.0 | 456.0 | 101 | 449.0 | 100 | 458.0 | 102 | 451.0 |
| 24.0 | 423.0 | 91 | 420.0 | 91 | 442.0 | 95 | 464.0 |
| 48.0 | 461.0 | 103 | 477.0 | 106 | 441.0 | 98 | 449.0 |
| 72.0 | 413.0 | 92 | 429.0 | 95 | 459.0 | 102 | 451.0 |
| 96.0 | 400.0 | 89 | 416.0 | 92 | 437.0 | 97 | 451.0 |
| 121.0 | 417.0 | 88 | 438.0 | 92 | 471.0 | 99 | 474.0 |
| 144.0 | 417.0 | 87 | 432.0 | 90 | 462.0 | 97 | 478.0 |
| 336.0 | 364.0 | 86 | 372.0 | 88 | 414.0 | 98 | 423.0 |
| 504.0 | 352.0 | 84 | 368.0 | 87 | 395.0 | 94 | 421.0 |

# 0 016 573

## TABLE XXXI
### LDH @ pH 8.5 in Human serum matrix

| Hour | 41°C | | 37°C | | 32°C | | −15°C |
|---|---|---|---|---|---|---|---|
| | A | A/D×100% | B | B/D×100% | C | C/D×100% | D |
| 0.0 | 488.0 | 100 | 488.0 | 100 | 488.0 | 100 | 488.0 |
| 4.0 | 476.0 | 98 | 487.0 | 100 | 478.0 | 98 | 488.0 |
| 8.0 | 504.0 | 105 | 529.0 | 110 | 531.0 | 110 | 482.0 |
| 24.0 | 438.0 | 89 | —N/A[1]— | | 476.0 | 97 | 491.0 |
| 48.0 | 436.0 | 88 | 471.0 | 95 | 482.0 | 97 | 498.0 |
| 72.0 | 478.0 | 92 | 505.0 | 97 | 521.0 | 100 | 520.0 |
| 96.0 | 428.0 | 81 | 472.0 | 90 | 504.0 | 96 | 527.0 |
| 172.0 | 405.0 | 78 | 451.0 | 86 | 470.0 | 90 | 522.0 |
| 316.0 | 353.0 | 69 | 404.0 | 79 | 449.0 | 87 | 514.0 |
| 484.0 | 309.0 | 59 | 406.0 | 78 | 484.0 | 93 | 521.0 |
| 1204.0 | 106.0 | 20 | 209.0 | 39 | 425.0 | 80 | 530.0 |

[1] N/A denotes not available.

Table XXXII compares the shelf life for each of the constituents listed in Tables XII—XXXI at a given temperature and time.

## TABLE XXXII

| Constituent | pH | Temp. (°C)/Time (Hr.) | % Recovery | |
|---|---|---|---|---|
| | | | Human albumin matrix | Human serum matrix |
| ALP | 6.5 | 41/144 | 78% | 55% |
| ALP | 8.5 | 41/96 | 97% | 64% |
| CK | 6.5 | 41/48 | 55% | 53% |
| CK | 8.5 | 41/24 | 13% | 2% |
| SGOT | 6.0 | 41/48 | 92% | 20% |
| SGOT | 8.5 | 41/72 | 98% | 50% |
| SGPT | 6.0 | 41/48 | 87% | 47% |
| SGPT | 8.5 | 41/24 | 79% | 56% |
| LDH | 6.5 | 41/~505 | 84% | 84% |
| LDH | 8.5 | 41/96 | 87% | 81% |

Table XXXII demonstrates that irrespective of pH enzymes tend to exhibit a markedly improved shelf life when stored in the human serum albumin matrix of the instant invention than when stored in the prior art human serum matrix of the composition of Maurukas I and Maurukas II, supra.

## Claims

1. An enzyme reference composition comprising:

(a) at least one enzyme constituent of known value;
(b) from 20 to 40 weight percent of at least one alkylene polyol having from 2 to 5 carbon atoms;
(c) from 3 to 8 grams per deciliter total protein present in a human serum albumin matrix; and
(d) from 60 to 80 weight percent water.

2. The composition of claim 1 having a pH of from 5.8 to 8.7.
3. The composition of claim 1 having a pH of from 6 to 8.5.
4. The composition of claim 1 having a pH of from 6 to 7.
5. The composition of claim 1 having a pH of from 6.4 to 6.6.
6. The composition of any of claims 1—4 or 5 comprising from 30 to 34 weight percent of said alkylene polyol, from 4 to 5 grams per deciliter of total protein, and from 66 to 70 weight percent water.
7. The composition of any of claims 1—4 or 5 wherein at least one of said enzymes is selected from a group consisting of acid phosphatase, aldolase, alkaline phosphatase, amylase, cholinesterase, creatine kinase, $\alpha$-glutamyl transpeptidase, $\alpha$-hydroxybutyric dehydrogenase, isocitric dehydrogenase, lactic dehydrogenase, leucine aminopeptidase, lipase, alanine amino transferase, and aspartate amino transferase.
8. The composition of any of claims 1—4 or 5 wherein said enzyme moiety comprises acid phosphatase, alkaline phosphatase, amylase, creatine kinase, aspartate amino transferase, alanine amino transferase, $\gamma$-glutamyl transpeptidase, and lactic dehydrogenase.

9. The composition of any of claims 1—4 or 5 comprising:

(a) from 0 to 350 International Units per liter alkaline phosphatase;
(b) from 0 to 20 International Units per liter acid phosphatase;
(c) from 0 to 400 International Units per liter amylase;
(d) from 0 to 600 International Units per liter creatine kinase;
(e) from 0 to 200 International Units per liter aspartate amino transferase;
(f) from 0 to 200 International Units per liter alanine amino transferase;
(g) from 0 to 300 International Units per liter $\gamma$-glutamyl transpeptidase; and
(h) from 0 to 500 International Units per liter lactic dehydrogenase wherein the enzyme activities are stated for 37°C.

10. The composition of any of claims 1—4 or 5 further comprising sodium, potassium, calcium, phosphorous, and magnesium.

11. The composition of any of claims 1—4 or 5 comprising:

(a) from 0 to 350 International Units per liter alkaline phosphatase;
(b) from 0 to 20 International Units per liter acid phosphatase;
(c) from 0 to 400 International Units per liter amylase;
(d) from 0 to 600 International Units per liter creatine kinase;
(e) from 0 to 200 International Units per liter aspartate amino transferase;
(f) from 0 to 200 International Units per liter alanine amino transferase;
(g) from 0 to 300 International Units per liter $\gamma$-glutamyl transpeptidase;
(h) from 0 to 500 International Units per liter lactic dehydrogenase;
(i) from 125 to 157 milliequivalents per liter sodium;
(j) from 3.5 to 4.5 milliequivalents per liter potassium;
(k) from 8.4 to 10.6 milligrams per deciliter calcium;
(l) from 1.6 to 2.4 milligrams per deciliter phosphorous;
(m) from 1.6 to 2.4 milligrams per deciliter magnesium; and
(n) from 88 to 112 milliequivalents per liter chloride;

wherein the enzyme activites are stated for 37°C.

12. The composition of any of claims 1—4 or 5 comprising:

(a) from 0 to 350 International Units per liter alkaline phosphatase;
(b) from 0 to 20 International Units per liter acid phosphatase;
(c) from 0 to 400 International Units per liter amylase;
(d) from 0 to 600 International Units per liter creatine kinase;
(e) from 0 to 200 International Units per liter aspartate amino transferase;
(f) from 0 to 200 International Units per liter alanine amino transferase;
(g) from 0 to 300 International Units per liter $\gamma$-glutamyl transpeptidase;
(h) from 0 to 500 International Units per liter lactic dehydrogenase;
(i) from 125 to 157 milliequivalents per liter sodium;
(j) from 3.5 to 4.5 milliequivalents per liter potassium;
(k) from 8.4 to 10.6 milligrams per deciliter calcium;
(l) from 1.6 to 2.4 milligrams per deciliter phosphorous;
(m) from 1.6 to 2.4 milligrams per deciliter magnesium; and
(n) from 88 to 112 milliequivalents per liter chloride;

wherein the enzyme activities are stated for 37°C; and wherein said acid phosphatase is derived from human seminal fluid, said alkaline phosphatase is derived from calf intestine; said amylase is derived from pig pancreas; said creatine kinase is derived from monkey muscle; said aspartate amino transferase and alanine amino transferase are derived from pig heart; said $\gamma$-glutamyl transpeptidase is derived from pig intestine; and said lactic dehydrogenase is derived from chicken heart.

**Patentansprüche**

1. Eine Mischung von Enzymen zu Bezugszwecken, bestehend aus:

(a) mindestens einem Enzymbestandteil bekannten Wertes;
(b) zwischen 20 und 40 Gewichtsprozent mindestens eines Alkylenpolyols, das zwischen 2 und 5 Kohlenstoffatome hat;

(c) zwischen 3 und 8 Gramm pro Deziliter Gesamtprotein, das in einer Matrix aus menschlichem Serumalbumin vorliegt;

(d) zwischen 60 und 80 Gewichtsprozent Wasser.

2. Mischung nach Anspruch 1 mit einem pH-Wert zwischen 5.8 und 8.7.

3. Mischung nach Anspruch 1 mit einem pH-Wert zwischen 6 und 8.5.

4. Mischung nach Anspruch 1 mit einem pH-Wert zwischen 6.0 und 7.0.

5. Mischung nach Anspruch 1 mit einem pH-Wert zwischen 6.4 und 6.6.

6. Mischung nach einem beliebigen Anspruchs 1 bis 4 oder 5, die zwischen 30 und 34 Gewichtsprozent des besagten Alkylenpolyiols zwischen 4 und 5 Gramm pro Deziliter Gesamtprotein und zwischen 66 und 70 Gewichtsprozent Wasser enthält.

7. Mischung nach einem beliebigen Anspruch 1 bis 4 oder 5, wobei mindestens eines der besagten Enzyme aus einer Gruppe ausgewählt wird, die besteht aus Saurer Phosphatase, Aldolase, Alkalischer Phosphatase, Amylase, Cholinesterase, Kreatin-Kinase, $\alpha$-Glutamyl-Transpeptidase, $\alpha$-Hydroxy-Buttersäure-Dehydrogenase, Isozitronensäure-Dehydrogenase, Milchsäure-Dehydrogenase, Leucin-Amino-Peptidase, Lipase, Alanin-Amino-Transferase und Bernsteinsäure-Amino-Transferase.

8. Mischung nach einem beliebigen Anspruch 1 bis 4 oder 5, wobei der besagte Enzymbestandteil besteht aus Saurer Phosphatase, Alkalischer Phosphatase, Amylase, Kreatin-Kinase, Bernsteinsäure-Amino-Transferase, Alanin-Amino-Transferase, $\gamma$-Glutamyl-Transpeptidase und Milchsäure-Dehydrogenase.

9. Mischung nach einem beliebigen Anspruch 1 bis 4 oder 5 bestehend aus:

(a) zwischen 0 und 350 Internationalen Einheiten pro Liter Alkalische Phosphatase;

(b) zwischen 0 und 20 Internationalen Einheiten pro Liter Saure Phosphatase;

(c) zwischen 0 und 400 Internationalen Einheiten pro Liter Amylase;

(d) zwishen 0 und 600 Internationalen Einheiten pro Liter Kreatin-Kinase;

(e) zwischen 0 und 200 Internationalen Einheiten pro Liter Bernsteinsäure-Amino-Transferase;

(f) zwischen 0 und 200 Internationalen Einheiten pro Liter Alanin-Amino-Transferase;

(g) zwischen 0 und 300 Internationalen Einheiten pro Liter $\gamma$-Glutamyl-Transpeptidase;

(h) zwischen 0 und 500 Internationalen Einheiten pro Liter Milchsäure-Dehydrogenase, wobei die Enzymaktivitäten für die Temperatur von 37°C angegeben sind.

10. Mischung nach einem beliebigen Anspruch 1 bis 4 oder 5, die des weiterin Natrium, Kalium, Kalzium, Phosphor und Magnesium enthält.

11. Mischung nach einem beliebigen Anspruch 1 bis 4 oder 5, bestehend aus:

(a) zwischen 0 und 350 Internationalen Einheiten pro Liter Alkalische Phosphatase;

(b) zwischen 0 und 20 Internationalen Einheiten pro Liter Saure Phosphatase;

(c) zwischen 0 und 400 Internationalen Einheiten pro Liter Amylase;

(d) zwischen 0 und 600 Internationalen Einheiten pro Liter Kreatin-Kinase;

(e) zwischen 0 und 200 Internationalen Einheiten pro Liter Bernsteinsäure-Amino-Transferase;

(f) zwischen 0 und 200 Internationalen Einheiten pro Liter Alanin-Amino-Transferase;

(g) zwischen 0 und 300 Internationalen Einheiten pro Liter $\gamma$-Glutamyl-Transpeptidase;

(h) zwischen 0 und 500 Internationalen Einheiten Milchsäure-Dehydrogenase;

(i) zwischen 125 und 157 Mille-Äquivalent pro Liter Natrium;

(j) zwischen 3.5 und 4.5 Milli-Äquivalent pro Liter Kalium;

(k) zwischen 8.4 und 10.6 Milligramm pro Deziliter Kalzium;

(l) zwischen 1.6 und 2.4 Milligramm pro Deziliter Phosphor;

(m) zwischen 1.6 und 2.4 Milligramm pro Deziliter Magnesium;

(n) zwischen 88 und 112 Milli-Äquivalent pro Liter Chlorid;

wobei die Enzymaktivitäten für die Temperatur von 37°C angegeben sind.

12. Mischung nach einem beliebigen Anspruch 1 bis 4 oder 5, bestehend aus:

(a) zwischen 0 und 350 Internationalen Einheiten pro Liter Alkalische Phosphatase;

(b) zwischen 0 und 20 Internationalen Einheiten pro Liter Saure Phosphatase;

(c) zwischen 0 und 400 Internationalen Einheiten pro Liter Amylase;

(d) zwischen 0 und 600 Internationalen Einheiten pro Liter Kreatin-Kinase;

(e) zwischen 0 und 200 Internationalen Einheiten pro Liter Bernsteinsäure-Amino-Transferase;

(f) zwischen 0 und 200 Internationalen Einheiten pro Liter Alanin-Amino-Transferase;

(g) zwischen 0 und 300 Internationalen Einheiten pro Liter $\gamma$-Glutamyl-Transpeptidase;

(h) zwischen 0 und 500 Internationalen Einheiten pro Liter Milchsäure-Dehydrogenase;

(i) zwischen 125 und 157 Milli-Äquivalent pro Liter Natrium;

(j) zwischen 3.5 und 4.5 Milli-Äquivalent pro Liter Kalium;

(k) zwischen 8.4 und 10.6 Milligramm pro Deziliter Kalzium;

(l) zwischen 1.6 und 2.4 Milligramm pro Deziliter Phosphor;
(m) zwischen 1.6 und 2.4 Milligramm pro Deziliter Magnesium;
(n) zwischen 88 und 112 Milli-Äquivalent pro Liter Chlorid;

wobei die Enzymaktivitäten für die Temperatur von 37°C angegeben sind und wobei die besagte Saure Phosphatase aus menschlicher Sammenflüssigkeit, die besagte Alkalische Phosphatase aus Kalbsdarm, die besagte Amylase aus Schweinepankreas, die besagte Kreatin-Kinase aus Affenmuskel, die besagte Bernsteinsäure-Amino-Transferase und die besagte Alanin-Amino-Transferase aus Schweineherz, die besagte γ-Glutamyl-Transpeptidase aus Schweinedarm und die besagte Milchsäure-Dehydrogenase aus Hühnerherz gewonnen werden.

**Revendications**

1. Une composition de référence d'enzymes comprenant:

(a) au moins un constituant d'enzyme de valeur connue:
(b) de 20 à 40 pourcent de poids d'au moins un polyol alcène possédant de 2 à 5 atomes de carbone;
(c) de 3 à 8 grammes per décilitre de la protéine totale présente dans une matrice d'albumine de sérum humain; et
(d) de 60 à 80 pourcent de poids d'eau.

2. La composition de la revendication 1 ayant un pH de 5,8 à 8,7.
3. La composition de la revendication 1 ayant un pH de 6 à 8,5.
4. La composition de la revendication 1 ayant un pH de 6 à 7.
5. La composition de la revendication 1 ayant un pH de 6,4 à 6,6.
6. La composition d'une revendication quelconque 1—4 ou 5 comprenant de 30 à 34 pourcent de poids dudit polyol alcène, de 4 à 5 grammes par décilitre de la protéine totale, et de 66 à 70 pourcent de poids d'eau.

7. La composition d'une revendication quelconque 1—4 ou 5 dans laquelle au moins un desdits enzymes est sélectionné dans un groupe composé de phosphatase acide, aldolase, phosphatase alcaline, amylase, cholinestérase, kinase de créatine, α-glutamyle transpeptidase, déshydrogénase α-hydroxybutrique, déshydrogénase isocitrique, déshydrogénase lactique, leucimaminopeptidase, lipase, alanine-aminotransférase, et aspartate-aminotransférase.

8. La composition d'une revendication quelconque 1—4 ou 5 dans laquelle la moitié desdits enzymes comprend: phosphatase acide, phosphatase alcaline, amylase, kinase de créatine, asparte-aminotransférase, alanine-aminotransférase, γ-glutamyle transpeptidase et déshydrogénase lactique.
9. La composition d'une revendication quelconque 1—4 ou 5 comprenant:

(a) de 0 à 350 Unités Internationales par litre de phosphatase alcaline;
(b) de 0 à 20 Unités Internationales par litre de phosphatase acide;
(c) de 0 à 400 Unités Internationales par litre d'amylase;
(d) de 0 à 600 Unités Internationales par litre de kinase de créatine;
(e) de 0 à 200 Unités Internationales par litre d'aspartate-amino-transférase;
(f) de 0 à 200 Unités Internationales par litre d'alanine-aminotransférase;
(g) de 0 à 300 Unités Internationales par litre de γ-glutamyle transpeptidase; et
(h) de 0 à 500 Unités Internationales par litre de déshydrogénase lactique où les activités des enzymes sont indiquées pour 37°C.

10. La composition d'une revendication quelconque 1—4 ou 5 comprenant en outre de sodium, potassium, calcium, phosphore et magnésium.
11. La composition d'une revendication quelconque 1—4 ou 5 comprenant:

(a) de 0 à 350 Unités Internationales par litre de phosphatase alcaline;
(b) de 0 à 20 Unités Internationales par litre de phosphatase acide;
(c) de 0 à 400 Unités Internationales par litre d'amylase;
(d) de 0 à 600 Unités Internationales par litre de kinase de créatine;
(e) de 0 à 200 Unités Internationales par litre d'aspartate-aminotransférase;
(f) de 0 à 200 Unités Internationales par litre d'alanine-aminotransférase;
(g) de 0 à 300 Unités Internationales par litre de γ-glutamyle transpeptidase;
(h) de 0 à 500 Unités Internationales par litre de déshydrogénase lactique;
(i) de 125 à 157 milliéquivalents per litre de sodium;
(j) de 3,5 à 4,5 milliéquivalents par litre de potassium;
(k) de 8,4 à 10,6 milligrammes par décilitre de calcium;
(l) de 1,6 à 2,4 milligrammes par décilitre de phosphore;

(m) de 1,6 à 2,4 milligrammes par décilitre de magnésium; et
(n) de 88 à 112 milliéquivalents par litre de chlorure;

où les activités des enzymes sont indiquées pour 37°C.

12. La composition d'une revendication quelconque 1—4 ou 5 comprenant:

(a) de 0 à 350 Unités Internationales par litre de phosphatase alcaline;
(b) de 0 à 20 Unités Internationales par litre de phosphatase acide;
(c) de 0 à 400 Unités Internationales par litre d'amylase;
(d) de 0 à 600 Unités Internationales par litre de kinase de créatine;
(e) de 0 à 200 Unités Internationales par litre d'aspartate-aminotransférase;
(f) de 0 à 200 Unités Internationales par litre d'alanine-aminotransférase;
(g) de 0 à 300 Unités Internationales par litre de $\gamma$-glutamyle transpeptidase;
(h) de 0 à 500 Unités Internationales par litre de déshydrogénase lactique;
(i) de 125 à 157 milliéquivalents par litre de sodium;
(j) de 3,5 à 4,5 milliéquivalents par litre de potassium;
(k) de 8,4 à 10,6 milligrammes par décilitre de calcium;
(l) de 1,6 à 2,4 milligrammes par décilitre de phosphore;
(m) de 1,6 à 2,4 milligrammes par décilitre de magnésium; et
(n) de 88 à 112 milliéquivalents par litre de chlorure;

où les activités des enzymes sont indiquées pour 37°C; et où ladite phosphatase acide provient du liquide séminal humain, ladite phosphatase alcaline provient de l'intestin de veau, ladite amylase provient du pancréas de porc, ladite kinase de créatine provientdu muscle de singe, lesdites aspartate-aminotransférase et alanine-aminotransférase proviennent du coeur de porc, ladite $\gamma$-glutamyle transpeptidase provient de l'intestin de porc et ladite déshydrogénase lactique provient du coeur de poulet.